# EUROPEAN PATENT APPLICATION

(11) **EP 4 570 821 A1**
(43) Date of publication of application: **18.06.2025**
(21) Application number: 23215416.1
(22) Date of filing: 11.12.2023
(51) Int. Cl.: C07K 14/705, A23C 9/00, C12N 5/071

(54) **MILK PRODUCING CELLS**

(71) Applicant: Senara GmbH, 79108 Freiburg (DE)
(72) Inventor: DANNEWITZ, Svenja, 79112 Freiburg (DE); PROSSEDA, Philipp Paolo, 79112 Freiburg (DE)
(74) Representative: Prüfer & Partner mbB Patentanwälte · Rechtsanwälte

(57) **Abstract**

The present invention relates to a method for preparing and/or isolating a cell population, which is enriched in lactating cells This method comprises a step of providing milk or mammary tissue, selecting a cell population comprising lactating cells based on specific cell markers, and obtaining said cell population being enriched in lactating cells. The present invention further refers to a cell culture comprising said lactating cells in an amount of at least 65% based on the total cells of the cell culture, and wherein the lactating cells are characterized by a specifically defined cell marker status. The present invention further refers to a container comprising said cell culture, as well as to a method of in vitro milk production, comprising the steps of providing and cultivating the cell population of the present invention, and harvesting the milk product.

## Description

### Field of the invention

The present invention relates to a method for preparing and/or isolating a cell population, which is enriched in lactating cells This method comprises a step of providing milk or mammary tissue, selecting a cell population comprising lactating cells based on specific cell markers, and obtaining said cell population being enriched in lactating cells. The present invention further refers to a cell culture comprising said lactating cells in an amount of at least 65% based on the total cells of the cell culture, and wherein the lactating cells are characterized by a specifically defined cell marker status. The present invention further refers to a container comprising said cell culture, as well as to a method of in vitro milk production, comprising the steps of providing and cultivating the cell population of the present invention, and harvesting the milk product.

### Background of the invention

The demand for worldwide milk production continually increases in response to a range of factors, including global population growth and changing, i.e., westernization, of diets in Asian countries. Worldwide consumption of dairy is projected to increase by over $100B over the coming decade. This, in turn, is placing increasing pressure on natural resources, increasing animal welfare concerns, and leading to increased deforestation alongside greater production of greenhouse gases. For example, dairy consumption in the U.S. accounts for about 2% of the country's greenhouse gas emissions. To meet the worldwide demand, around 270 million dairy cows are farmed every day. Every component in the milk has been associated with an array of both positive and negative health effects.

Milk is a complex colloidal matrix that contains milk fat, lactose, and a number of different components, including naturally occurring hormones. The sensory and functional characteristics of milk stem from its micronutrient profile that has proven costly and difficult to replicate. In general, the gross composition of cow's milk per 100 g is 87.7 g water, 3.2 g protein, 3.9 g fat,4.8 g carbohydrate (i.e., the sugar form of lactose), 14 mg cholesterol, and 120 mg calcium.

The composition of the milk varies depending on the species (cow, goat, sheep), breed (Holstein, Jersey), the animal's feed, and the stage of lactation. Studies have shown that the fat and protein content of milk is based on the breed of cattle. Holsteins have the lowest fat and protein content, while Jersey and Guernsey breeds have the highest fat and protein content. Even within a single herd, the milk protein can range from 1.57% to 4.66%, with an average of 3.05%; while the milk fat ranges from 1.77% to 5.98%, with an average of 3.76%. The food source, temperature, humidity, and seasonality all played important roles to the variation of milk quality in milk production.

The milk's quality is - amongst other - depending on the presence of naturally occurring hormones found in milk, and other foreign agents, such as antibiotics and pharmaceutics. In particular other foreign agents are often present in industrial-scale milk arising from the need to treat an entire herd for infections during their milk production (i.e., chronic udder infections).

In order to avoid potential issues (e.g., health problems) that go along with milk consumption, the idea came up to substitute animal-based milk by plant-based milk and milk products. Milk from plants such as almond, soy, cashew, and oat lack the naturally occurring hormones found in animal-based milk and are becoming increasingly popular as a good source of protein without raising the issues of dairy intolerance that many people experience. However, the plant-based milks from these alternative sources tend not to be as effective in providing the functionality of animal-based milk that translates to production of other dairy products such as cheese, butter, and yogurt. Furthermore, the increased popularity of plant-based milks has brought to light other sustainability concerns, for example, the widespread deforestation that has been employed to expand soy production, or the large amounts of water required for almond production in areas experiencing long-term drought.

Hence, milk and dairy products still constitute a significant component of the human diet, providing important sources of protein, vitamins, and minerals. For many people, dairy is the easiest way to obtain supplementary nutrients to keep the heart, muscles, and bones healthy and functioning properly. However, the milk quality related issues discussed above have plagued the dairy industry for many years and urgently need a solution. In this connection, for example WO 2022/054053 A1 refers to a method for *in vitro* milk production by employing an array of mammary organoids seeded on tertiary-branched, resilient duct scaffolding.

However, still, there is an apparent need for improvements to milk production technology, e.g., in terms of improved methods for milk production, driven by several factors including the current unsustainable approach to producing milk through cattle farming (inefficient use of land and agricultural resources, greenhouse gas production, and more); the lack of comparable alternatives to milk; and the limited sources of consistent quality and high-quality milk.

### Summary of the invention

In the present invention, a method for preparing and/or isolating a specific cell population which is enriched in lactating cells, is provided. This method comprises a step of providing milk or mammary tissue, a step of selecting a cell population that is enriched in the desired lactating cells, and a step of obtaining the cell population being enriched in the lactating cells. The step of selecting the cell population comprising the lactating cells is based on the identification of the desired lactating cells based on at least one cell marker which is selected from a specific group of cell markers. It has been surprisingly found in the present invention that the lactating cells can be isolated based on this specific group of cell markers; and finally, the thus-isolated (or identified, respectively) lactating cells can be used in an in vitro method of preparing milk. Being able to prepare milk by using the specifically enriched cell culture comprising the targeted cell population provides multiple advantages: For example, the subject-matter of the present invention represents a powerful tool for producing milk and dairy products while at the same time the production process is subject to controlled conditions, e.g., in terms of purity and safety. Moreover, as it is a cell culture comprising specifically defined lactating cells, the milk production process is also improved in terms of environmental aspects.

Accordingly, the present invention provides the following aspects, subject-matter and preferred embodiments which, respectively taken alone or in combination, contribute to providing improved technical effects and to solving the afore-mentioned object of the invention:
1. Method for preparing and/or isolating a cell population being enriched in lactating cells, wherein the method comprises the following steps:
   (a) providing (a-1) milk or (a-2) a mammary tissue;
   (b) selecting a cell population comprising lactating cells (and further non-lactating cells) from the milk or from the mammary tissue, wherein the lactating cells to be enriched are selected on the basis of at least one cell marker selected from the group consisting of:
      CD49f, EpCAM/CD326, Cytokeratin 7, Cytokeratin 8, Cytokeratin 10, Cytokeratin 18, Cytokeratin 19, Muc1/CD227, CD24, CD133/ PROM1, Her2, CD117, CD166, CD282, CD340, Claudin 4, C-MET, ER, PR, VDR;
      and optionally further one or more selected from CD9, CD47, CD54, CD59, CD95, CD164, CD49b, CD66, CK8/18, CK19, MUC1, EPCAM, CD13, CD15s/CD73/CLA, CD166, CD227, CD340, CD10, CD90, CD200, CD29, CD142, CD271, CK5, CK14, CK17, SMA, V1M, CD34/CD39/CD140b, CD49e, Claudin-4, E-Cadherin, EGFR, Integrin α9β1, Nectin-4; and
   (c) obtaining a cell population being enriched in lactating cells, wherein the cell population comprises lactating cells in an amount of at least 70% based on the total amount of cells being present in the cell population.
2. Method according to item 1, wherein in step (b) the lactating cells to be enriched are selected on the basis of at least one cell marker selected from the group consisting of:
   CD49f, EpCAM/CD326, Cytokeratin 7, Cytokeratin 8, Cytokeratin 10, Cytokeratin 18, Cytokeratin 19, Muc1/CD227, CD24, CD133/ PROM1, Her2, CD117, CD166, CD282, CD340, Claudin 4, C-MET, ER, PR, VDR;
   and optionally further one or more selected from CD9, CD47, CD54, CD59, CD95, CD164, CD49b, CD66, CK8/18, CK19, MUC1, EPCAM, CD13, CD15s/CD73/CLA, CD166, CD227, CD340, CD10, CD90, CD200, CD29, CD142, CD271, CK5, CK14, CK17, SMA, V1M, CD34/CD39/CD140b, and CD49e.
3. Method according to item 1 or 2, wherein in step (b) the lactating cells to be enriched are selected on the basis of at least one cell marker selected from the group consisting of CD49f, EpCAM, CD24, CD29, CD10, and CK18,
   and optionally further one or more selected from CD9, CD47, CD54, CD59, CD95, CD164, CD49b, CD66, CD24, CK8/18, CK19, MUC1, EPCAM, CD13,
   CD15s/CD73/CLA, CD166/CD227/CD340, CD10, CD90, CD200, CD29, CD10, CD142, CD271, CK5, CK14, CK17, SMA, V1M, CD34/CD39/CD140b, and CD49e.
4. Method according to item 1, wherein in step (b) the lactating cells to be enriched are selected on the basis of at least one cell marker selected from the group consisting of:
   CD49f, CD326 (EpCAM), CD24, CD133/PROM1, CD24, Muc1, CD29, CD227, CD166, CD10, CD282, CD340;
   preferably selected from the group consisting of CD49f, EpCAM, CD24, and CD29, CD10;
   and optionally further
   Claudin 4, CD9, CD47, CD54, CD59, CD95, CD49b, CD13, CD166, CD227, CD340, CD90, CD200, CD142, CD271, CD34, CD39, CD140b, E-Cadherin, Claudin 4, EGFR, Integrin α9β1, Nectin-4, Her2, CD117, and CD49e.
5. Method according to item 4, wherein in step (b) the lactating cells to be enriched are further selected on the basis of at least one cell marker selected from the group consisting of CK7, CK8, CK10, CK18, CK19, smooth muscle actin (SMA), vimentin (V1M), Hormone receptors such as ER (estrogen receptor), PR (progesterone receptor), and VDR (vitamin D receptor).
6. Method according to items 1, 2, 3, and 5, wherein in step (b) the lactating cells to be enriched are further selected on the basis of at least one surface marker selected from the group consisting of
   CD24 positive, and CD29 negative or CD29 low;
   and optionally being further CD49f positive or CD49f negative; EpCAM positive or EpCAM negative; CK18 positive and CD10 negative.
7. Method according to any one of the preceding items, wherein in step b) the lactating cells to be enriched are further characterized by a receptor status of being estrogen receptor alpha (ERα) positive and/or progesterone receptor (PR) positive.
8. Method according to any one of the preceding items, wherein in step b) the lactating cells to be enriched are characterized based on a level of expression of one or more of the cell surface markers and/or ERα or PR.
9. Method according to any one of the preceding items, wherein in step (b) the lactating cells to be enriched are selected based on the following surface marker pattern:
   (i) CD24 medium expression;
      CD49f positive; and
      EpCAM negative;
      and/or
   (ii) CD24 high;
      CD49f negative;
      CD29 low;
      EpCAM positive;
      CD10 negative;
      and/or
   (iii) CD24 medium expression;
      CD49f negative;
      CD29 low;
      EpCAM positive,
      optionally wherein the cell population has less than 30 % cells that exhibit the following surface maker pattern:
   (iv) CD24 negative;
      CD49f high;
      CD29 high;
      EpCAM negative
      CD10 high.
10. Method according to any one of the preceding items, wherein the step (b) of selecting the cell population to be enriched comprises a flow cytometry technology or other positive and negative selection mammary gland cell isolation techniques such as techniques based on immunomagnetism or other affinity related cell selection techniques.
11. Method according to any of the preceding items, wherein the milk of step (a) is cow milk, preferably obtained from a cow in a status range between day 30 and day 250 of lactation, more preferably between day 30 and day 150 of lactation, and even more preferably between day 90 and day 150 of lactation.
12. Method according to any one of the preceding items, wherein the mammary tissue is mammalian breast tissue, i.e., human or animal breast tissue.
13. Method according to any one of the preceding items, wherein the mammary tissue is a biopsy of a mammary gland.
14. Method according to any one of the preceding items, wherein the cell population comprises lactating cells in an amount of at least 70%, preferably at least 80%, more preferably in an amount of at least 90%, more preferably in an amount of at least 95%, and most preferred in an amount of at least 98%, based on the total cells of the cell population.
15. Method according to any one of the preceding items, wherein the cell population obtained in step (c) is a second cell population, and wherein step (b) comprises at least one pre-selection step (b-0), thereby first obtaining a first cell population, wherein the ratio of lactating cells to non-lactating cells in the first cell population after the pre-selection step is enhanced compared to the ratio of lactating cells to non-lactating cells prior to the pre-selection step.
16. Method according to any one of the preceding items, wherein the pre-selection step (b-0) comprises a step involving one or more of the following techniques:
   a) a cell separation step which removes cell debris, or which separates cells based on size, such as Forward Scatter detection (FSC) and/or Side Scatter detection (SSC);
   b) a cell separation step based on immunomagnetism, such as a marker-based immunomagnetic cell separation step, and/or
   c) a cell separation step based on flow cytometry techniques, such as marker-based fluorescence activated cell sorting (FACS).
17. Method according to any one of the preceding items, wherein the pre-selection step (b-0) b) and/or (c) represents a positive and/or negative selection step, preferably a negative selection step.
18. Method according to any one of the preceding items, wherein step (b) comprises
   (b-1) at least one pre-selection step being an immunomagnetic cell separation step or FACS separation step for obtaining a first cell population partly enriched in lactating cells; and
   (b-2) a selection step being a flow cytometry step for obtaining the cell population further enriched in lactating cells in step (c), preferably a fluorescence-activated cell sorting (FACS) analysis, or other technique using these markers.
19. Method according to any one of the preceding items, further comprising a step (d) of culturing and optionally scaling up the cell population enriched in lactating cells obtained in step (c).
20. Method according to any one of the preceding items, further comprising a step of immortalizing the cell population obtained in step (c) or step (d).
21. Cell culture comprising a cell population, wherein this cell population comprises lactating cells in an amount of at least 70% based on the total cells of the cell culture, and wherein lactating cells are characterized based on the presence or absence of at least one cell marker selected from the group as defined in step (b) of items 1 to 9.
22. Cell culture according to item 21, wherein this cell population comprises lactating cells in an amount of at least 70% based on the total cells of the cell culture, and wherein lactating cells are characterized based on the presence or absence of at least one cell marker selected from the group consisting of CD49f, EpCAM/CD326, Cytokeratin 7, Cytokeratin 8, Cytokeratin 10, Cytokeratin 18, Cytokeratin 19, Muc1/ CD227, CD24, CD133/ PROM1, Her2, CD117, CD166, CD282, CD340, Claudin 4, C-MET, ER, PR ,VDR;
   and optionally further CD9, CD47, CD54, CD59, CD95, CD164, CD49b, CD66, CD24, CK8/18, CK19, MUC1, EPCAM, CD13, CD15s/CD73/CLA, CD166/CD227/CD340, CD10, CD90, CD200, CD29, CD10, CD142, CD271, CK5, CK14, CK17, SMA, V1M, CD34/CD39/CD140b, and CD49e.
23. Cell culture according to item 21 or 22, wherein the lactating cells are characterized based on the expression or non-expression of cell marker being selected from the group consisting of CD49f, EpCAM, CD24, CD29, CD10, and CK18.
24. Cell culture according to any one of items 21 to 23, wherein progenitor and the lactating cells are characterized by a cell marker status being CD24 positive;
   and optionally being further CD49f positive or CD49f negative; EpCAM positive or EpCAM negative; CK18 positive and CD10 negative.
25. Cell culture according to any one of items 21 to 24, further characterized by a receptor status of being estrogen receptor alpha (ERα) positive and/or progesterone receptor (PR) positive.
26. Cell culture according to any one of items 21 to 25, wherein the lactating cells are characterized based on a level of expression of one or more of the cell markers and/or the ERα or PR.
27. Cell culture according to any one of items 21 to 26, wherein the cell population comprises lactating cells in an amount of at least 75%, preferably at least 80%, more preferably at least 90%, even more preferably in an amount of at least 95%, and most preferred in an amount of at least 98%, based on the total cells of the cell culture.
28. Cell culture according to any one of items 21 to 27, wherein the lactating cells are further characterized by one or more of the following cell markers:
   CD326, Cytokeratin 7, Cytokeratin 8, Cytokeratin 10, Cytokeratin 19, CD227 (also referred to as Muc1), CD133 (also referred to as PROM1), Her2, CD117, CD166, CD282, CD340, Claudin 4, C-MET, ER, PR, VDR.
29. Cell culture according to any one of items 21 to 28, wherein the cell population comprises cells that exhibit the following cell marker pattern:
   (i) CD24 medium expression;
      CD49f positive; and
      EpCAM negative;
      and/or
   (ii) CD24 high;
      CD49f negative;
      CD29 low;
      EpCAM positive;
      CD10 negative;
      and/or
   (iii) CD24 medium expression;
      CD49f negative;
      CD29 low;
      EpCAM positive,
      optionally wherein the cell population has less than 30% cells that exhibit the following surface maker pattern:
   (iv) CD24 negative;
      CD49f high;
      CD29 high;
      EpCAM negative
      DC10 high.
30. Cell culture according to any one of items 21 to 29, wherein the cells of the cell population are primary cells or immortalized cells.
31. Container comprising the cell culture as defined in any one of items 21 to 30 in a suitable cell culture medium.
32. Container according to item 31, wherein the container has a volume of 1 ml to 1000 l.
33. Method of in vitro milk production, comprising the following steps:
   (i) providing the cell population as defined in any one of items 21 to 30, or providing the cell population being enriched in lactating cells by the method as defined in any one of items 1 to 20;
   (ii) cultivating the cell population of step (i) under suitable conditions; and
   (iii) harvesting milk product.
34. Method according to item 33, wherein step (ii) comprises transferring the cell population comprising the lactating cells into a suitable apparatus.
35. Method according to item 33 or 34, wherein step (i) includes firstly preparing a master batch of the cell population enriched in the lactating cells, and secondly transferring at least a part of the master batch into one or more sub-batches, whereupon steps (ii) and (iii) follow from the one or more sub-batch(es).
36. Method according to anyone of items 33 to 35, wherein step (i) includes firstly preparing a master batch, in which the cells are cultured and expanded, subsequently optionally stored.

### Brief description of the drawings

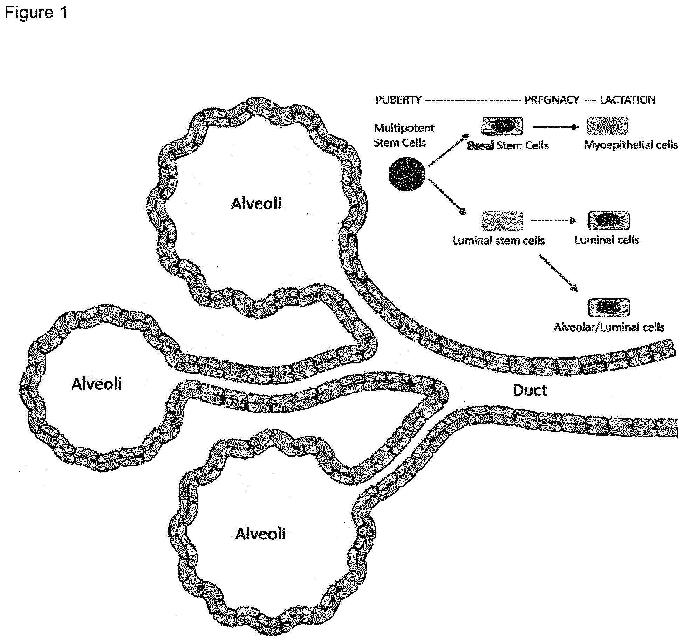

Figure 1: This figure shows basal versus luminal cells. Particularly, figure 1 is a visual representation of mammary duct structures and cell types found in the mammary glands.

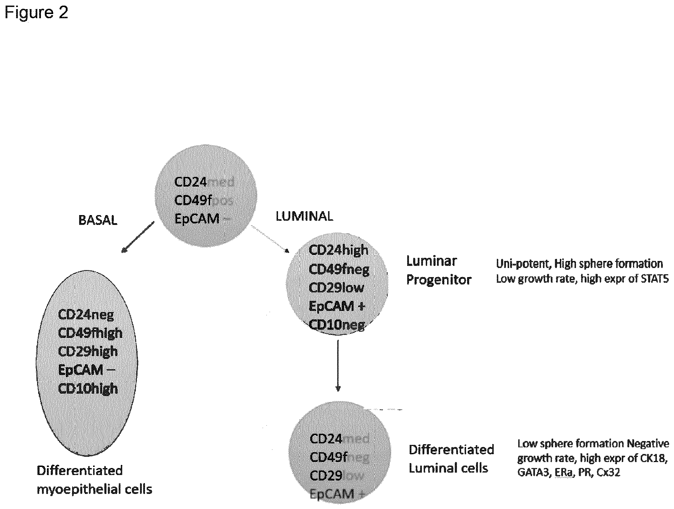

Figure 2: This figure shows one possible combination from the panel of cell surface markers for mammary gland cell isolation. In order to identify cell populations, different rates of expressions of surface markers are shown in figure 2, which make it possible to discriminate between mature and progenitor luminal/basal cells in mammary cell of different species, including human, cow, goat, mouse, and other mammalians.

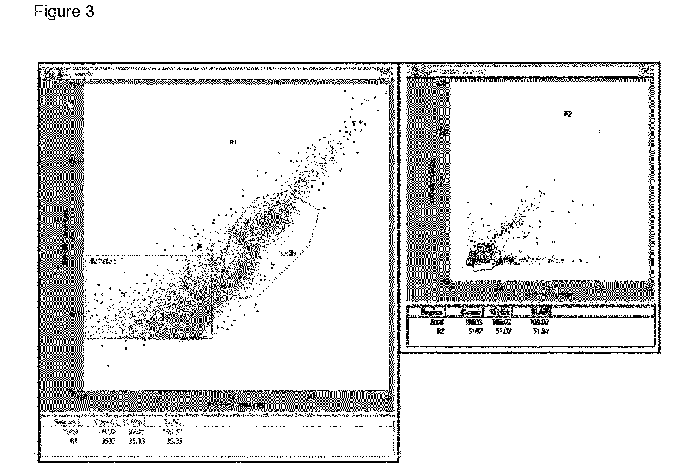

Figure 3: This figure shows an example of a pre-selection (pre-sorting) step, carried out on a sample of fresh cow milk. In this example, an initial flow-cytometry (pre-sorting) step has been performed to isolate mammalian cells from the rest of the biomass in the fresh milk. A variety of Laser combinations without cell marker staining can be used in this simple pre-sorting step, such as:
   488 nm Laser (or other relative Laser) for Forward Scatter FSC detection
   488nm Laser (or other relative Laser) for Side Scatter SSC detection
   The forward scatter is located in line with the laser intercept and is typically considered a measure of the relative cell size. The side scatter is typically located perpendicular to the laser beam intercept and is used to measure the relative complexity of the cell.
   The fresh milk is run through the flow cytometer and the content is detected using the FFS (site) and SSC (complexity). Using specific gating strategies such as (but not limited to) shown in this figure, the cell part of the fresh milk is isolated from the rest of the biomass and sorted out using cell sorting technique of the flow cytometer into a new sample consisting only of milk derived mammalian cells in suspension.
   However, it is also possible to perform a pre-sorting step that is not a flow cytometry step, but e.g., a presorting step based on immunomagnetism, such as magnetic cell sorting.

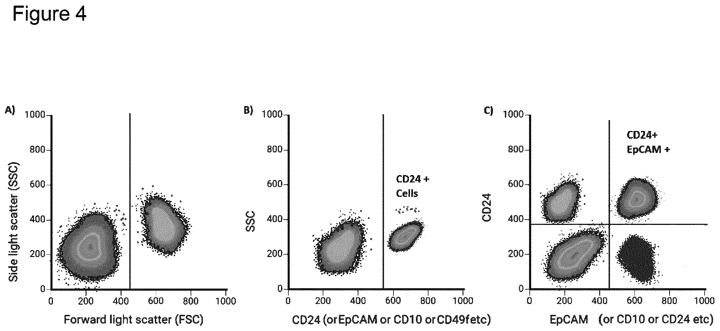

Figure 4: This figure shows the analytical approach employed for examining the stained cell populations via flow cytometry was as follows:
   Figure. 4 A: To establish the analysis of individual viable cells, a gating strategy was implemented. This strategy included the exclusion of cell doublets based on FSC-H/A and SSC-H/A parameters. Additionally, the use of propidium iodide enabled the differentiation between living and non-viable cells.
   Figure 4 B: Each individual staining involving markers of interest such as but not limited to anti-CD49f, -CD10, -CD24, and -EpCAM antibodies was performed on viable cell populations.
   Figure 4 C: One possible gating strategy adopted for the analysis of multi-stained cell populations was delineated.
   Initially, CD24-positive cells were singled out, and subsequently, co-immunostained with markers of interest such as but not limited to anti CD24 and EpCAM, CD24 and CD10, as well as CD49f and CD24 or other combinations.
   To ensure the removal of aggregates, the flow cytometry process incorporates doublet exclusion by utilizing FSC-H/A and SSC-H/A parameters. Furthermore, for the differentiation between live and non-viable cells, propidium iodide (PI) staining is applied. This technical approach is crucial for the individualized analysis of each cell-surface marker on live cells.
   The method for assessing the expression of other cellular markers, e.g., as outlined in Fig. 2, within a stained population (in this case, the CD49f-positive cells), is detailed in Figure 4 A to C.
   For instance, the CD49f-positive cell subset can be gated to facilitate the analysis of co-immunostained populations, such as CD49f and EpCAM, among others. Both CD49f and EpCAM are cell-surface markers known for their association with cell adhesion and are frequently employed for characterizing mammary gland cells and assessing their level of maturation.
Figure 5: This figure shows a comparison of positive and negative selection. Fig. 5 A shows positive versus negative magnetic selection. Fig. 5 B shows that the selection can also be done without magnet and magnetic beads using a surface coated directly with antibodies for selection.

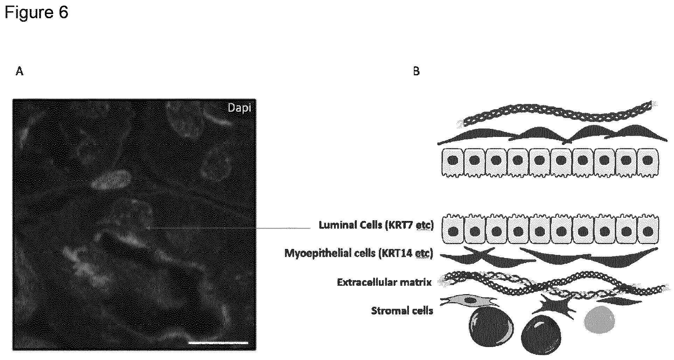

Figure 6: This figure shows the organization of epithelial cells, which can be analyzed by immunofluorescence using different markers.
Figure 6 A: This figure shows epithelial cells stained with different markers for caracterization and visualized via fluorescence microscopy at 60X magnification.
Figure 6 B: This figure shows a model of epithelium in lactating mammary gland, representing the epithelial layer with the different cell types. Colonies of cells expressing myoepithelial markers exhibit a compact arrangement of small polygonal cells with diminished cytoplasm. In contrast, colonies of cells expressing luminal markers present as spindle-shaped cells with distinct nuclei and an elongated cytoplasm. Furthermore, some of these colonies include only a small number of cells characterized by a highly extended cytoplasm and irregular shapes.
Figure 7: This figure shows first attached cells after sorting.

### Detailed description of the invention

The invention is described below in further detail by embodiments, without being limited thereto.

### Method for preparing and/or isolating a cell population being enriched in lactating cells, cell marker selection group A

In one aspect, the present invention refers to a method for preparing and/or isolating, preferably isolating, a cell population being enriched in lactating cells, wherein the method comprises the following steps: (a) providing (a-1) milk or (a-2) a mammary tissue; (b) selecting a cell population comprising lactating cells (and further non-lactating cells) from the milk or from the mammary tissue, wherein the lactating cells to be enriched are selected on the basis of at least one cell marker selected from the group consisting of CD49f, EpCAM/CD326, Cytokeratin 7, Cytokeratin 8, Cytokeratin 10, Cytokeratin 18, Cytokeratin 19, Muc1/CD227, CD24, CD133/ PROM1, Her2, CD117, CD166, CD282, CD340, Claudin 4, C-MET, ER, PR, VDR; and optionally further one or more selected from CD9, CD47, CD54, CD59, CD95, CD164, CD49b, CD66, CK8/18, CK19, MUC1, EPCAM, CD13, CD15s/CD73/CLA, CD166, CD227, CD340, CD10, CD90, CD200, CD29, CD10, CD142, CD271, CK5, CK14, CK17, SMA, V1M, CD34/CD39/CD140b, CD49e, Claudin-4, E-Cadherin, EGFR, Integrin α9β1, Nectin-4; and (c) obtaining a cell population being enriched in lactating cells, wherein the cell population comprises lactating cells in an amount of at least 70% based on the total amount of cells being present in the cell population.

While this marker selection represents the general group of cell markers based on which the lactating cells to be enriched are selected (isolated), in a preferred embodiment, the lactating cells are selected on the basis of at least one or more cell markers selected from the groups described below.

In the present invention, in step (b), the selection on the basis of the at least one cell marker may mean a positive selection ("sorting in" for further processing with step (c)) or a negative selection ("sorting out" for not further processing with step (c)), and/or on the basis of high, medium or low marker presence on the cell surface (respectively for "sorting in" or "sorting out" for being further processed or not with step (c)), in relation to the respectively mentioned marker.

A skilled person is able to differentiate between cell populations on the basis of certain cell markers. For example, when applying flow cytometry techniques, such as FACS analysis, cell populations can be distinguished based on the expression of certain cell surface markers, i.e., marker(s) that is (are) expressed on the cell's surface. A certain cell is classified as being "positive" regarding a specific marker, if this marker is expressed on the cell surface. In general, in FACS technology, highly specific antibodies are labelled with fluorescent conjugates and thus FACS allows to simultaneously collect data in, and sort a biological sample by a nearly limitless number of different parameters. Whether a certain cell population expresses a certain cell marker or not, and also to assess whether the expression-status or presence of this cell marker is negative, medium, or high, can be assessed by comparing the fluorescent or light scattering characteristics of the cell population to be analysed with the fluorescent or light scattering characteristics of a negative control cell population (that is, a cell population that is known to not express the cell marker in question), and/or (preferably and) with the fluorescent or light scattering characteristics of a positive control (that is, a cell population that is known to expresses the cell marker in question). Based on the fluorescent or light scattering characteristics, specific gates can be set. For example, all cells of an analysed cell population that express a certain cell marker are "positive" regarding this marker and can be gated for further analysis (e.g., "sorting in"). Whether or not the analysed cell population comprises cells that express a certain cell marker (i.e., these cells are "positive" regarding this cell marker), and whether the expression of this cell marker is "medium" or "high" or "low", can be determined by a skilled person based on a comparison of the fluorescent or light scattering characteristics of the positive control population, negative control population, and the population in question.

In a preferred embodiment, in step (b), the lactating cells to be enriched are selected on the basis of at least one cell marker selected from the group consisting of CD49f, CD326 (EpCAM), CD24, CD133/PROM1, CD24, Muc1, CD29, CD227, CD166, CD10, CD282, CD340; preferably selected from the group consisting of CD49f, EpCAM, CD24, and CD29, CD10; and optionally further Claudin 4, CD9, CD47, CD54, CD59, CD95, CD49b, CD13, CD166, CD227, CD340, CD90, CD200, CD142, CD271, CD34, CD39, CD140b, E-Cadherin, Claudin 4, EGFR, Integrin α9β1, Nectin-4, Her2, CD117, and CD49e.

It is further preferred, that in step (b), the lactating cells to be enriched are further (additionally) selected on the basis of at least one cell marker selected from the group consisting of CK7, CK8, CK10, CK18, CK19, smooth muscle actin (SMA), vimentin (V1M), Hormone receptors such as ER (estrogen receptor), PR (progesterone receptor), and VDR (vitamin D receptor).

### Method for preparing and/or isolating a cell population being enriched in lactating cells, cell marker selection group B

As already described above with regard to cell marker selection group A, the present invention refers to a method for preparing and/or isolating, preferably isolating, a cell population being enriched in lactating cells, wherein the method comprises the following steps: (a) providing (a-1) milk or (a-2) a mammary tissue; (b) selecting a cell population comprising lactating cells (and further non-lactating cells) from the milk or from the mammary tissue, wherein the lactating cells to be enriched are selected on the basis of at least one cell marker selected from the group consisting of CD49f, EpCAM/CD326, Cytokeratin 7, Cytokeratin 8, Cytokeratin 10, Cytokeratin 18, Cytokeratin 19, Muc1/CD227, CD24, CD133/ PROM1, Her2, CD117, CD166, CD282, CD340, Claudin 4, C-MET, ER, PR, VDR; and optionally further one or more selected from CD9, CD47, CD54, CD59, CD95, CD164, CD49b, CD66, CK8/18, CK19, MUC1, EPCAM, CD13, CD15s/CD73/CLA, CD166, CD227, CD340, CD10, CD90, CD200, CD29, CD10, CD142, CD271, CK5, CK14, CK17, SMA, V1M, CD34/CD39/CD140b, CD49e, Claudin-4, E-Cadherin, EGFR, Integrin α9β1, Nectin-4; and (c) obtaining a cell population being enriched in lactating cells, wherein the cell population comprises lactating cells in an amount of at least 70% based on the total amount of cells being present in the cell population.

While this marker selection represents the general group of cell markers based on which the lactating cells to be enriched are selected (isolated), in a preferred embodiment, the lactating cells are selected on the basis of at least one or more cell markers selected from the group consisting of CD49f, EpCAM/CD326, Cytokeratin 7, Cytokeratin 8, Cytokeratin 10, Cytokeratin 18, Cytokeratin 19, Muc1/CD227, CD24, CD133/ PROM1, Her2, CD117, CD166, CD282, CD340, Claudin 4, C-MET, ER, PR, VDR; and optionally further one or more selected from CD9, CD47, CD54, CD59, CD95, CD164, CD49b, CD66, CK8/18, CK19, MUC1, EPCAM, CD13, CD15s/CD73/CLA, CD166, CD227, CD340, CD10, CD90, CD200, CD29, CD142, CD271, CK5, CK14, CK17, SMA, V1M, CD34/CD39/CD140b, and CD49e.

In a further preferred embodiment, the cell markers of step (b) are selected from the group consisting of CD49f, EpCAM, CD24, CD29, CD10, and CK1; and optionally further one or more selected from CD9, CD47, CD54, CD59, CD95, CD164, CD49b, CD66, CD24, CK8/18, CK19, MUC1, EPCAM, CD13, CD15s/CD73/CLA, CD166/CD227/CD340, CD10, CD90, CD200, CD29, CD10, CD142, CD271, CK5, CK14, CK17, SMA, V1M, CD34/CD39/CD140b, and CD49e.

In an even more preferred embodiment, the cell markers of step (b) are selected from the group consisting of CD24 positive and CD29 negative; and optionally being further CD49f positive or CD49f negative; EpCAM positive or EpCAM negative; CK18 positive and CD10 negative.

These cell markers are indicative of mammary epithelial cells, particularly of progenitor and mature luminal mammary epithelial cells.

### Method for preparing and/or isolating a cell population being enriched in lactating cells, cell marker selection groups A and B

The following preferred groups of cell markers apply to cell marker selection groups A and B.

In a preferred embodiment, in the method of the present invention, in step b) the lactating cells to be enriched are further characterized by a receptor status of being estrogen receptor alpha (ERα) positive and/or progesterone receptor (PR) positive.

It is also possible that in the method of the present invention, in step b) the lactating cells to be enriched are characterized based on a level of expression of one or more of the cell surface markers and/or ERα or PR.

In an even more preferred embodiment, in the method of the present invention, in step (b) the lactating cells to be enriched are selected based on the following surface marker pattern:
(i) CD24 medium expression;
   CD49f positive; and
   EpCAM negative;
   and/or
(ii) CD24 high;
   CD49f negative;
   CD29 low;
   EpCAM positive;
   CD10 negative;
   and/or
(iii) CD24 medium expression;
   CD49f negative;
   CD29 low;
   EpCAM positive,
   optionally wherein the cell population has less than 30 % cells that exhibit the following surface maker pattern:
(iv) CD24 negative;
   CD49f high;
   CD29 high;
   EpCAM negative
   DC10 high.

Surface marker pattern (i) is characteristic for progenitor cells of the basal and the luminal cells. Surface marker pattern (ii) is characteristic for luminar progenitor cells, surface marker pattern (iii) is characteristic for differentiated luminal cells, and surface marker pattern (iv) is characteristic for differentiated myoepithelial cells (which are basal cells).

In further preferred embodiments, the cell population has less than 20%, preferably less than 15%, more preferably less than 10%, even more preferably less than 7%, and most preferred less than 2% cells that exhibit the surface marker pattern (iv).

In step (b), a cell population comprising lactating cells (the cells which are to be enriched) is selected. This selection step can be carried out by applying any suitable selection method that is known to a skilled person. For example, step (b) of selecting the cell population to be enriched comprises a flow cytometry technology or other positive and negative selection cell isolation techniques such as techniques based on immunomagnetism or other affinity related cell selection techniques.

In step (a) of the method of the present invention, milk of a mammary tissue is provided. In a preferred embodiment, the milk is cow milk, preferably obtained from a cow in a status range between day 30 and day 250 of lactation, more preferably between day 30 and day 150 of lactation, and even more preferably between day 90 and day 150 of lactation. In these status ranges, the milk yield is comparably high. Table 1 shows the timing of maximum cell yield. As can be seen, milk yield is maximum between 90 to 140 days of lactation, an dCK19 gene expression is highest 150 days of lactation:

**Table 1: Flow cytometry analysis of single cell suspensions using anti-CD24, -EpCAM, - CD10 and -CD49f antibodies, mean fluorescence of each staining in %.**

| | | | | |
|---|---|---|---|---|
| | | | | |

| **Days of Lactation** | **30** | **90** | **150** | **250** |
|---|---|---|---|---|
| **Milk yield (Kg/d)** | 30 | 34 | 32 | 20 |
| | | | | |

| **Surface markers % (flow cyt analysis)** | | | | |
|---|---|---|---|---|
| **CD49f+** | **48** | **44** | **38** | **29** |
| **CD24+** | **5.5** | **5.7** | **3.9** | **3.2** |
| **EpCAM+** | **4.4** | **6.9** | **6.2** | **4.5** |
| **CD10+** | **4** | **5.7** | **3.9** | **4.7** |

It is also possible that mammary tissue is provided as source for the lactating cells to be enriched. Preferably, the mammary tissue is mammalian breast tissue, i.e., human or animal breast tissue, wherein the mammary tissue is a biopsy of a mammary gland.

In a preferred embodiment, the cell population being enriched in lactating cells comprises lactating cells in an amount of at least 70%, preferably at least 80%, more preferably in an amount of at least 90%, more preferably in an amount of at least 95%, and most preferred in an amount of at least 98%, based on the total cells of the cell population.

Selecting the cell population to be enriched (step b) comprises e.g., a flow cytometry technology or other positive and negative selection mammary gland or cow milk cell isolation techniques such as techniques based on immunomagnetism or other affinity related cell selection techniques. For example, using flow cytometry in in conjunction with specific cell-surface markers offers a highly sensitive method for the assessment of cell populations and subpopulations within the mammary gland, but also within the cow milk. Notably, the expression of cluster of differentiation (CD) molecules, such as CD24 (heat-stable antigen) and CD49f (integrin alpha-6), has been widely adopted for the identification of mammary gland epithelial cells.

In the lactating mammary gland, the continuous process of self-renewal relies on two fundamental mechanisms: cell proliferation and programmed cell death, known as apoptosis. Progenitor cells take centre stage in this biological phenomenon, as they actively partake in both proliferation and the generation of differentiated cells responsible for milk synthesis. Subsequently, these differentiated cells are eliminated from the mammary epithelium through apoptosis.

Further characterization is performed through IF and PCR profiling of genes of interest.

For example, mammary cells can be characterized via gene expression profile (PCR, "polymerase chain reaction"). The following genes are some of the most noteworthy genes that are expressed in milk secreting cells:

| | |
|---|---|
| **CSN2:** | Casein Beta (β) gene, which encodes for beta-casein. |
| **CSN1S1** | Casein Alpha S1 (αS1) gene, specifically its protein product in the context of milk. |
| **BTN1A1** | is associated with the transport of lipids and the regulation of milk fat production. |
| **LALBA** | "Lactalbumin, Alpha," and it is a gene that encodes for alpha-lactalbumin, a protein found in the milk of mammals. |
| **STATS** | The expression of STAT5 in lactating mammalian cells is a crucial aspect of the molecular mechanisms that govern milk production and secretion (response effect to prolactin) |
| **KRT18** | Keratin 18 can be observed in the epithelial cells of the mammary alveoli and ducts. |
| **GAPDH** | Control |
| **ALDH1** | Not species restricted. ALDH high cells always have luminal features. ALDH activity can be used to distinguish luminal progenitors from both myoepithelial clonogenic progenitors and mammary stem cells with in vivo regenerative activity. |

Table 2 shows suitable primer sequences for RT (reverse transcriptase)-PCR:

**Table 2: Primer sequences for RT-PCR.**

| Gene Name | Pair version 1 | Pair version 2 | Pair version 3 |
|---|---|---|---|
| **CSN1S1** | F-CAAAGTACCCCAGCTGGAAA R-ACCAGGCACCAGATGGATAG | F- ATGAAACTTCTCATCCTTACCTGTCTT R- CCAATATCCTTGCTCAGTTCATT | |
| **CSN1S2** | F-AGCTCTCCACCAGTGAGGAA R-GCAAGGCGAATTTCTGGTAA | F-CGAGGACAAAGCAAACATGA R-AGGTTCTCCTTGCTGGGATT | |
| **CSN2** | F CAGAAAGCAGTGCCCTATCC R-GCCATATTTCCAAGTCGCAGT | F-AGGAACAGCAGCAAACAG3 R-TTTCCAGTCGCAGTCAAT3 | F GTGAGGAACAGCAGCAAACA R-AGGGAAGGGCATTTCTTTGT |
| **BTN1A1** | F-AGGCAGGACGAAAACTACGA R-CCTGTGAGTTCGCCACTGTA | F-TGTGTTGCTGCTGATAGAGTGT R-CCTCCAAGTTCCTTTATGGGAT | F-CAGTCACTGATGGGCTT R-GACAGTGGGATCTCCTTTG |
| **LALBA** | F-TACTGGTTGGCCCATAAAGC R-CAAGGGGGTACAAAGAAGCA | F- CTCTCTGCTCCTGGTAGGCAT R- GTGAGGGTTCTGGTCGTCTT | F- CTTACTTCACTCCCGTTAGCA R- GACAGAAGCAGCAAAGACAGC |
| ***STAT5*** | F- GTGAAGCCACAGATCAAGCA R- TCGAATTCTCCATCCTGGTC | | |
| ***GAPDH*** | F- GGCAAGTTCAACGGCACA3 R- TCTTCTGGGTGGCAGTGATGG | F- TGGAAAGGCCATCACCATCT R- CCCACTTGATGTTGGCAG | |

Fig. 1 is a visual representation of mammary duct structure and cell types found in the mammary glands. The mammary gland is a crucial anatomical structure in mammals, responsible for lactation and milk production. Comprising glandular tissue, it undergoes significant changes during puberty, pregnancy, and lactation cycles. Myoepithelial Cells surround the alveoli and ducts and aid milk ejection through contraction during breastfeeding. Cells involved in milk production are the Epithelial Cells which are divided in two main groups: Luminal cells and basal cells.

### Basal Cells:

Basal cells are found at the base of the ducts, contributing to the maintenance and regeneration of the glandular tissue. Typically, they are found in the basal or outer layer of the mammary gland ducts. They are in close contact with the basement membrane, which separates the glandular tissue from the surrounding stroma. Basal cells are often referred to as myoepithelial cells because they have contractile properties and are involved in the contraction of the mammary gland ducts to help push milk toward the duct during breastfeeding. They also provide structural support to the ducts. Basal cells are characterized by the expression of specific markers, such as smooth muscle actin (SMA) and cytokeratin 14 (CK14). These markers are associated with their contractile and structural functions. Basal cells are less differentiated and are considered precursor cells. They have the potential to give rise to both basal and luminal cells. Basal cells are less responsive to hormonal changes, such as those associated with the menstrual cycle or pregnancy.

### Luminal cells:

Luminal cells are situated on the inner layer of the mammary gland ducts. They line the luminal space within the ducts and are in direct contact with the lumen where milk is produced and transported. Luminal cells are primarily responsible for producing and secreting milk components, including proteins and lipids. They play a central role in milk synthesis and transport. Luminal cells express markers like cytokeratin 18 (CK18) and estrogen receptor (ER), reflecting their role in milk production and hormone responsiveness. Luminal cells are highly responsive to hormonal fluctuations and undergo significant changes in response to pregnancy and lactation.

In order to identify a cell population that is enriched in lactating cells, cell markers such as cell surface markers or gene expression of suitable marker genes can be analyzed. Cell surface markers are present on the surface of cells and can e.g. be used for cell sorting techniques based on flow cytometry, such as FACS analysis. Suitable cell surface markers are for example:
CD49f, CD326 (EpCAM), CD24, CD133/PROM1, CD24, Muc1, CD29, CD227, CD166, CD10, CD282, CD340, Claudin 4, CD9, CD47, CD54, CD59, CD95, CD49b, CD13, CD166, CD227, CD340, CD90, CD200, CD142, CD271, CD34, CD39, CD140b, E-Cadherin, Claudin 4, EGFR, Integrin α9β1, Nectin-4, Her2, CD117.

Other cell markers which are not cell surface markers can be used for a cell characterization after selection of cells based on flow cytometry techniques. Markers like cytokeratins ((CK7, CK8, CK10, CK18, CK19), smooth muscle actin (SMA), vimentin (V1M), and other intracellular proteins are typically not used as surface markers in flow cytometry since they are present within the cell and require permeabilization for detection. Similarly, hormone receptors such as ER (estrogen receptor), PR (progesterone receptor), and VDR (vitamin D receptor) are not surface markers but rather intracellular proteins that are typically assessed through immunohistochemistry (IHC) or other specific assays.

Hence, in general, additional cell markers can be used for Immunohistochemistry (IHC), Immunofluorescence, Western Blotting, Flow Cytometry, PCR and RT-PCR and other techniques which might use these markers for isolating and characterizing the desired cells, e.g., mammary cells from human and animal tissue or milk. The most common markers to identify luminal epithelial cells are CK8, CK18, CK19, whereas CK5, CK14, smooth muscle actin, and vimentin are the most frequently used markers to detect basal myoepithelial cells.

The most common markers to identify luminar breast epithelial cells are: Cytokeratin 7, Cytokeratin 8, Cytokeratin 10, Cytokeratin 18, Cytokeratin 19, Her2, Claudin 4, C-MET, ER, PR, VDR, GATA3; with Cytokeratin 8 and cytokeratin 18 being the most prominent luminar breast epithelial marker.

The most common markers to identify basal/myoepithelial breast epithelium cells are: Cytokeratin 14, SMA, Cytokeratin 5, Vimentin, Connexin 43, Integrin α9β1, p63, P-Cadherin.

Figure 2 shows surface markers and their diverse rate expression; for example, based on these surface markers and their relative expression, it is possible for a skilled person to discriminate between mature and progenitor luminal/basal cells in mammary species, including human, goat, mouse, and other mammalians.

In step (b) of the method of the present invention, a cell population comprising lactating cells (and further non-lactating cells) from the milk or from the mammary tissue is selected, based on the cell markers disclosed herein. It is possible that this selection step (b) is not only one single selection step, but it is possible, and it may be of advantage, to carry out one or more pre-selection (presorting) steps. After having carried out the one or more pre-selection steps, the ratio of lactating cells to non-lactating cells in the first cell population after the pre-selection step is enhanced compared to the ratio of lactating cells to non-lactating cells prior to the pre-selection step. It is also possible that the selection step is not a positive selections tep, but a negative election step, that is, cells that are selected based on a specific cell marker status, are discarded.

For example, if a pre-selection step (b-0) is carried out, the cell population obtained in step (c) is a second cell population, thereby first obtaining a first cell population, wherein the ratio of lactating cells to non-lactating cells in the first cell population after the pre-selection step is enhanced compared to the ratio of lactating cells to non-lactating cells prior to the pre-selection step.

The pre-selection step (b-0) can comprise a step involving one or more of the following techniques:
a) a cell separation step which removes cell debris, or which separates cells based on size, such as Forward Scatter detection (FSC) and/or Side Scatter detection (SSC);
b) a cell separation step based on immunomagnetism, such as a marker-based immunomagnetic cell separation step, and/or
c) a cell separation step based on flow cytometry techniques, such as marker-based fluorescence activated cell sorting (FACS).

In the method of the present invention, the pre-selection step (b-0) b) and/or (c) represents a positive and/or negative selection step, preferably a negative selection step.

In a further preferred embodiment, the negative selection step comprises FACS analysis and/or an immunomagnetic cell separation step.

For example, an initial flow cytometry (presorting step) can be performed to isolate mammalian cells from the rest of the biomass in the fresh milk: A variety of laser combinations that are known to a skilled person, without cell marker staining, can be used in this simple pre-sorting step, such as:
488 nm Laser (or other relative laser) for Forward Scatter FSC detection;
488nm Laser (or other relative laser) for Side Scatter SSC detection.

The forward scatter is located in line with the laser intercept and is typically considered a measure of the relative cell size. The side scatter is typically located perpendicular to the laser beam intercept and is used to measure the relative complexity of the cell.

For example, the fresh milk which is for example provided in step (a) of the inventive method is run through the flow cytometer and the content is detected using the FFS (site) and SSC (complexity). Using specific gating strategies such as (but not limited to) shown in figure 3, the cell part of the fresh milk is isolated from the rest of the biomass and sorted out using cell sorting technique of the flow cytometer into a new sample consisting only of milk derived mammalian cells in suspension. The "cells" that are gated in the rectangle on the left side of figure 3 represent e.g. cell debris and hence this fraction of the sample is discarded. The content of the gate on the right side of figure 3 represents cells. These cells are further processed/analysed.

If, for example, a mammary tissue (such as a mammary biopsy) or milk is used in the inventive method, the following steps can be carried out:
- Obtaining biological cells from mammary biopsy digestions or directly isolating them from fresh milk.
- Preparing the cells for flow cytometry analysis using a customized F-Buffer containing 2% bovine serum albumin.
- Loading each analysis tube with a standardized quantity of 500,000 cells.
- Incubating the cells in a dark environment at a controlled temperature of 4°C for a defined duration of 30 minutes.
- Applying one or a combination of the required antibodies for cellular labeling for the cell markers disclosed herein, with a gating strategy Panel such as: Fluorescein isothiocyanate (FITC) anti-rat IgG1 CD49f (α6 integrin) or others in the lists disclosed elsewhere herein.
- Utilizing isotype controls for each antibody to establish specific fluorescence signals and eliminate non-specific background signals.
- Following the labelling process, subjecting the cells to centrifugation at 400 × g and re-suspending them in a predetermined volume of the F-Buffer (0.3 mL).
- Conducting multi-staining by employing the four antibodies to identify specific cellular populations.
- Employing Propidium Iodide (PI) to distinguish live cells within the sample.
- Gating the PI-stained population with regards to forward-scattered light (FSC) and side-scattered light (SSC) for the exclusion of non-viable cells and to delineate cell granularity, size, and doublets, making necessary adjustments in height and photomultiplier voltage.
- Performing flow cytometry analysis on a dataset containing 30,000 events, specifically single cells, with flow cytometer.
- Expressing the results in the form of percentages, with a focus on dot plot analysis.

In Fig. 4, flow cytometry isolation of specific cell types from milk or tissue as well as an example of a possible gating strategy on accordingly labelled cells, is depicted: To ensure the removal of aggregates, the flow cytometry process incorporates doublet exclusion by utilizing FSC-H/A and SSC-H/A parameters. Furthermore, for the differentiation between live and non-viable cells, propidium iodide (PI) staining is applied. This technical approach is crucial for the individualized analysis of each cell-surface marker on live cells.

The method for assessing the expression of other cellular markers, as outlined elsewhere herein, within a stained population (in this case, the CD24-positive cells), is detailed in figure 4. For instance, as can be seen in fig. 4, the CD24-positive cell subset can be gated to facilitate the analysis of co-immunostained populations, such as CD24 and EpCAM, among others. Both CD24 and EpCAM are cell-surface markers known for their association with cell adhesion and are frequently employed for characterizing mammary gland cells and assessing their level of maturation.

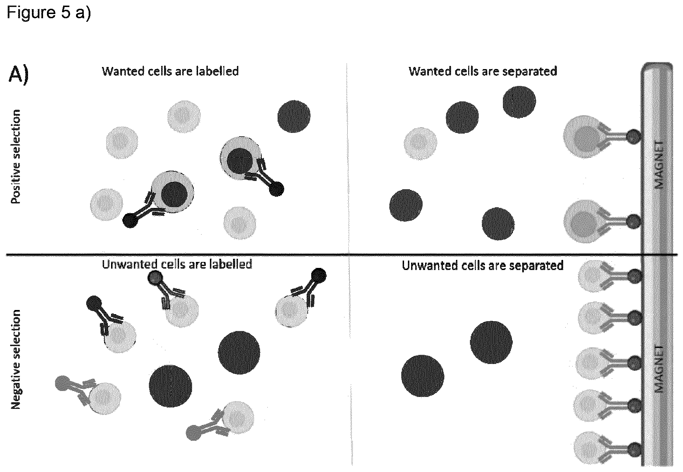

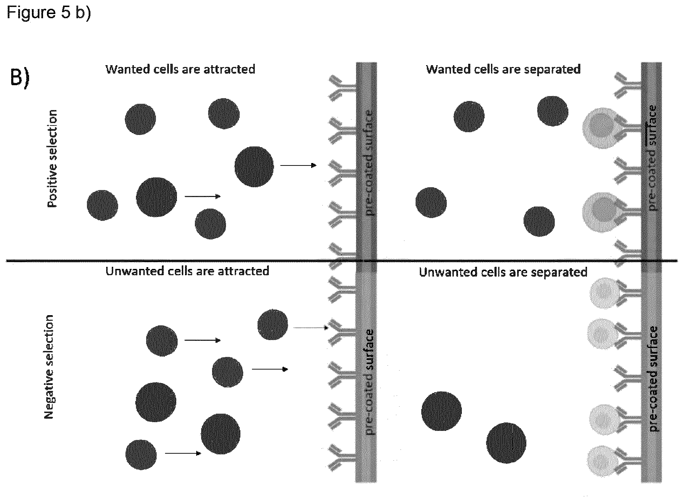

It is possible to carry out a selection of cells based on positive selection and based on negative selection. The principle of positive and negative selection is depicted in figure 5.

Positive selection immunomagnetic cell separation techniques entail the direct labeling of target cells (which are lactating cells in the present invention) with an antibody or ligand designed to recognize a specific cell surface protein. This antibody or ligand is affixed to a magnetic particle, facilitating the retention of labeled cells in the final isolated fraction following sample incubation in a magnetic field. Alternatively, surfaces pre-coated with antibodies specific to the cells of interest can also serve for positive selection. Negative selection immunomagnetic cell separation methodologies include the labeling of undesired cell types with antibodies or ligands directed against specific cell surface proteins. These antibodies or ligands are attached to magnetic particles, enabling the removal of labeled unwanted cells from the final isolated fraction through sample incubation in a magnetic field. As the desired cells are not specifically targeted by antibodies or ligands, they remain unbound by the particles. Alternatively, surfaces pre-coated with antibodies specific to the cells of interest can also serve for negative selection.

Positive and negative selection can be employed, using the selection markers for mammary gland cell isolation in different species.

For example, in the method of the present invention, the markers disclosed elsewhere herein can be used for the isolation of a milk producing mammary gland cells through immunomagnetic cell separation. Notably, the markers disclosed herein can be employed in both positive and negative selection methods such as positive and negative immunomagnetic selection.

### Positive Immunomagnetic Cell Selection:

The Markers disclosed elsewhere herein, e.g., the cell markers disclosed in fig. 2, can be utilized for positive selection, wherein cells expressing the target antigen are specifically isolated. One way of doing this is to conjugate these markers to magnetic beads which allows for the efficient isolation of the desired cell population (fig. 5 A). This can also be done using pre-coated surface coated directly with antibodies of interest for positive selection (fig. 5 B)

### Negative Immunomagnetic Cell Selection:

Furthermore, the disclosed markers are applicable to negative selection strategies, enabling the isolation of cells by selectively depleting unwanted populations. E.g., markers shown in fig. 2 can be conjugated to beads and can be employed to remove cells expressing specific antigens, resulting in the isolation of the target subset (fig. 5 A). This can also be done using pre-coated surface coated directly with antibodies of interest for negative selection (fig. 5 B).

The versatility of the markers shown in fig. 2 in both positive and negative cell selection methods enhance the utility and applicability of the disclosed invention. This dual functionality provides a powerful tool for the precise isolation of target cell populations. Importantly, negative selection can be used for quick pre-enrichment of target cells prior to Fluorescence-activated cell sorting (FACS). The isolation of rare cell types through fluorescence-activated cell sorting (FACS) may necessitate high initial volumes and extended sort times. However, the duration of cell sorting can be significantly reduced by employing immunomagnetic cell separation to pre-enrich the target cells. Negative selection is often the preferred approach due to its quick process, high recovery rates, and the absence of labeling on the cells of interest. This flexibility enables to utilize fluorophore-tagged antibodies against specific cell surface markers during FACS.

In addition, indirect positive selection can be useful for the isolation of less common or difficult to isolate cell types. When dealing with the isolation of uncommon cells lacking specific, readily available cell separation kits markers, it is possible to employ the technique of indirect positive selection. In the case of immunomagnetic separation variant, this method achieves cell separation by utilizing commonly accessible secondary antibodies to bind magnetic particles to the target cells, previously labeled by primary antibodies. This approach opens the possibility of isolating almost any cell type through the application of indirect positive selection. Complex cell types will necessitate a combination of both positive and negative selection methods to achieve effective purification.

In a preferred embodiment, step (b) comprises
(b-1) at least one pre-selection step being an immunomagnetic cell separation step or FACS separation step for obtaining a first cell population partly enriched in lactating cells; and
(b-2) a selection step being a flow cytometry step for obtaining the cell population further enriched in lactating cells in step (c), preferably a fluorescence-activated cell sorting (FACS) analysis, or other technique using these markers.

It is additionally preferred that the method of the present invention further comprises a step (d) of culturing and optionally scaling up the cell population enriched in lactating cells obtained in step (c).

The culturing and scaling up can be carried out by applying suitable cell culture media and apparatus that are known to a skilled person.

Moreover, it is possible to carry out a further step of immortalizing the cell population obtained in step (c) or step (d). Immortalizing is a common technique and can be carried out by a skilled person according to a standard procedure.

The present invention further refers to a cell culture comprising a cell population, wherein this cell population comprises lactating cells in an amount of at least 70% based on the total cells of the cell culture, and wherein lactating cells are characterized based on the presence or absence of at least one cell marker selected from the group as defined in step (b) described in more detail elsewhere herein.

In a preferred embodiment, the cell population comprises lactating cells in an amount of at least 70% based on the total cells of the cell culture, and wherein lactating cells are characterized based on the presence or absence of at least one cell marker selected from the group consisting of CD49f, EpCAM/CD326, Cytokeratin 7, Cytokeratin 8, Cytokeratin 10, Cytokeratin 18, Cytokeratin 19, Muc1/ CD227, CD24, CD133/ PROM1, Her2, CD117, CD166, CD282, CD340, Claudin 4, C-MET, ER, PR, VDR;
and optionally further CD9, CD47, CD54, CD59, CD95, CD164, CD49b, CD66, CD24, CK8/18, CK19, MUC1, EPCAM, CD13, CD15s/CD73/CLA, CD166/CD227/CD340, CD10, CD90, CD200, CD29, CD10, CD142, CD271, CK5, CK14, CK17, SMA, V1M, CD34/CD39/CD140b, and CD49e.

In a further preferred embodiment, the lactating cells of the cell culture are characterized based on the expression or non-expression of cell marker being selected from the group consisting of CD49f, EpCAM, CD24, and CD29, CD10, and CK18.

Particularly, the progenitor and lactating cells are characterized by a cell marker status being CD24 positive and CD29 negative;
and optionally being further CD49f positive or CD49f negative; EpCAM positive or EpCAM negative; CK18 positive and CD10 negative.

It is additionally possible that the cell culture of the present invention is further characterized by a receptor status of being estrogen receptor alpha (ERα) positive and/or progesterone receptor (PR) positive.

It is also possible that the lactating cells can be characterized based on a level of expression of one or more of the cell markers described elsewhere herein, and/or the ERα or PR.

The level of expression can be determined by any suitable method that is known to a skilled person; preferably, the method is based on flow cytometry, preferably the method is fluorescence-activated cell sorting (FACS). Alternatively, or in combination, the level of expression can be determined by gene expression or receptor determination.

In a preferred embodiment, the cell population of the cell culture of the present invention comprises lactating cells in an amount of at least 75%, preferably at least 80%, more preferably at least 90%, even more preferably in an amount of at least 95%, and most preferred in an amount of at least 98%, based on the total cells of the cell culture.

In a further preferred embodiment, the lactating cells that are present in the cell culture are further characterized by one or more of the following cell markers:
CD326, Cytokeratin 7, Cytokeratin 8, Cytokeratin 10, Cytokeratin 19, CD227 (also referred to as Muc1), CD133 (also referred to as PROM1), Her2, CD117, CD166, CD282, CD340, Claudin 4, C-MET, ER, PR, VDR.

In a particular preferred embodiment, the cell population that is present in the cell culture of the present invention comprises cells that exhibit the following cell maker pattern:
(i) CD24 medium expression;
   CD49f positive; and
   EpCAM negative;
   and/or
(ii) CD24 high;
   CD49f negative;
   CD29 low;
   EpCAM positive;
   CD10 negative;
   and/or
(iii) CD24 medium expression;
   CD49f negative;
   CD29 low;
   EpCAM positive,
   optionally wherein the cell population has less than 30% cells that exhibit the following surface maker pattern:
(iv) CD24 negative;
   CD49f high;
   CD29 high;
   EpCAM negative
   DC10 high.

Preferably, the cell population comprises less than 20%, preferably less than 15%, more preferably less than 10%, even more preferably less than 7%, and most preferred less than 2% cells that exhibit the surface marker pattern (iv).

The cells of the cell population can be primary cells or immortalized cells; preferably, the cells are immortalized cells.

The present invention also refers to a container comprising the cell culture of the present invention, in a suitable cell culture medium. A skilled person knows which cell culture medium is suitable for the respective cell culture.

In a preferred embodiment, the container is a container that is suitable for the respective cell culture, depending on the further use of the cell culture. The container can have any suitable volume, preferably the container has a volume of 1 ml to 1000 l.

The present invention also refers to a method of in vitro milk production, comprising the following steps:
(i) providing the cell population of the present invention, or providing the cell population being enriched in lactating cells by the method of the present invention;
(ii) cultivating the cell population of step (i) under suitable conditions; and
(iii) harvesting the milk product.

Again, a skilled person knows which medium is suitable for cultivating the cell population. For example, a medium used for culturing the cells for use in in vitro milk production is preferably FCS- and hormone-free (e.g., except for prolactin).

In a preferred embodiment, step (ii) comprises transferring the cell population comprising the lactating cells into a suitable apparatus. For example, a suitable apparatus is an apparatus that provides an environment (culture conditions) that enables the cell population to produce in vitro milk.

In an embodiment, step (i) includes firstly preparing a master batch of the cell population enriched in the lactating cells, and secondly transferring at least a part of the master batch into one or more sub-batches, whereupon steps (ii) and (iii) follow from the one or more sub-batch(es).

It is also possible that step (i) includes firstly preparing a master batch, in which the cells are cultured and expanded, subsequently optionally stored.

### Examples

### 1. Dissociation of cells (only for tissue)

The dissociated cells obtained from this protocol can be utilized for various downstream applications such as cell culture, flow cytometry, sorting, gene expression analysis, or any other experimental techniques requiring isolated cells from animal tissue.

### Materials:

- Animal tissue (e.g., mammary tissue)
- Dulbecco's Modified Eagle Medium (DMEM) or other suitable cell culture medium
- Collagenase or other tissue-dissociating enzyme
- Trypsin-EDTA solution
- Hank's Balanced Salt Solution (HBSS) or phosphate-buffered saline (PBS)
- Sterile petri dishes or culture plates
- Scalpel or scissors
- Forceps
- Centrifuge tubes
- Cell strainer (70 µm)

### Procedure:

1. Preparation:
   - Obtain fresh animal tissue and place it in a dish containing cold HBSS or PBS.
   - Rinse the tissue thoroughly to remove excess blood and debris.
2. Tissue Dissection:
   - Using sterile instruments (scissors or scalpel), finely mince the tissue into small pieces (1-2 mm) in a sterile dish or on a clean surface.
   - Transfer the minced tissue pieces into a sterile tube or container.
3. Enzymatic Digestion:
   - Add an appropriate volume of enzyme solution (e.g., collagenase) to the minced tissue. The concentration and incubation time depend on the enzyme used and the tissue type.
   - Incubate the tissue-enzyme mixture at an appropriate temperature (usually 37°C) for a specified duration, typically 30 minutes to several hours, depending on the tissue and enzyme used. Gently agitate or mix the tube periodically during incubation.
4. Neutralization of Enzyme Activity:
   - After the incubation period, add an equal or excess volume of DMEM or culture medium containing FBS to neutralize the enzymatic activity and inhibit further digestion.
   - Centrifuge the cell suspension at a low speed (e.g., 300-500 × g) for a few minutes to pellet the cells.
5. Cell Resuspension:
   - Remove the supernatant and resuspend the cell pellet in a suitable volume of fresh culture medium (DMEM, RPMI, etc.) supplemented with FBS or other additives.
6. Optional: Trypsin Treatment for Further Dissociation:
   - If required, further dissociate cell aggregates by treating the cell suspension with a mild Trypsin-EDTA solution for a short duration (e.g., 5-10 minutes at 37°C).
   - Inactivate trypsin activity by adding excess culture medium with FBS.
7. Cell Straining:
   - Pass the cell suspension through a 70 µm cell strainer to obtain a single-cell suspension and remove any remaining tissue debris or aggregates.
8. Cell Counting and Viability Assessment:
   - Use a hemocytometer or an automated cell counter to determine cell concentration and assess cell viability using trypan blue or a similar dye exclusion method.

### 2. Isolation of Epithelial Cells from Tissue/Milk using FACS

### Materials:

- Tissue sample (e.g., mammary tissue) or milk
- Dulbecco's Phosphate-Buffered Saline (DPBS) without calcium and magnesium
- Trypsin-EDTA solution
- Cell culture medium (appropriate for epithelial cells)
- Fetal bovine serum (FBS)
- Antibodies against epithelial cell markers (e.g., EpCAM, cytokeratins)
- Fluorescence-activated cell sorter (FACS) machine
- 70 µm cell strainer
- Centrifuge tubes
- Pipettes and tips
- Ice bucket

### Procedure:

1. Tissue/Milk Sample Preparation:
   - For tissue: Minced the tissue sample into small pieces or use milk directly.
   - If using tissue, enzymatically digest it with trypsin-EDTA solution at 37°C for 30-60 minutes. For milk, skip this step.
2. Cell Suspension Preparation:
   - Filter the digested tissue through a 70 µm cell strainer to obtain a single-cell suspension.
   - Centrifuge the suspension at 300-400 × g for 5 minutes to pellet cells.
3. Cell Surface Marker Staining:
   - Resuspend the cell pellet in DPBS and count viable cells using a hemocytometer or automated cell counter.
   - Aliquot 1-5 million cells per staining tube (adjust according to the FACS machine capacity).
   - Add the appropriate volume of antibody cocktail against epithelial cell markers (e.g., EpCAM, cytokeratins) and incubate on ice for 15-30 minutes, protecting from light.
4. Washing and Resuspension:
   - Wash the stained cells by adding excess DPBS and centrifuge at 300-400 × g for 5 minutes.
   - Discard the supernatant and resuspend the cell pellet in cell culture medium supplemented with FBS.
5. FACS Sorting:
   - Set up the FACS machine according to manufacturer protocols, ensuring proper instrument calibration and control settings.
   - Filter the stained and resuspended cells through a 35 µm nozzle into the FACS sample tube placed on ice.
   - Sort the cells based on their fluorescence signals corresponding to the epithelial markers. Collect sorted cells in a sterile collection tube containing fresh cell culture medium.
6. Post-Sorting Analysis:
   - Analyze a portion of sorted cells by microscopy or flow cytometry to confirm the purity and viability of isolated epithelial cells.
   - Culture the sorted cells in appropriate cell culture conditions for downstream experiments or analysis.

### 3. Culturing Sorted Epithelial Cells

### Materials:

- Culture vessels (T-flasks, culture plates, etc.) suitable for epithelial cell growth
- Cell culture medium suitable for epithelial cells (e.g., DMEM/F-12 supplemented with growth factors, antibiotics, and FBS, customized mix of growth factors for primary cells.
- Coating material (if required)
- Sterile pipettes, tips, and centrifuge tubes
- Trypsin-EDTA solution
- Hemocytometer or automated cell counter
- Microscope

### Procedure:

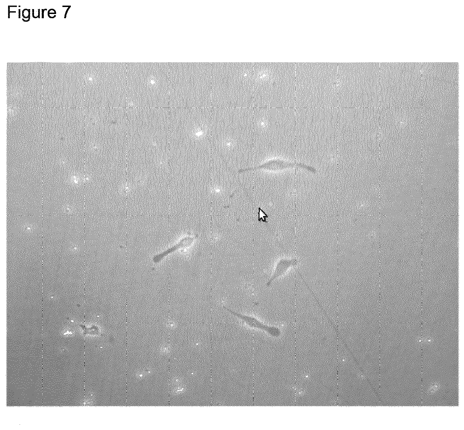

1. Preparation of Culture Vessels:
   - Coat culture vessels if necessary (e.g., coating with collagen, fibronectin) according to the manufacturer's recommendations. Alternatively, use pre-coated plates/flasks suitable for epithelial cell growth.
2. Culture Medium Preparation:
   - Prepare the culture medium by adding appropriate growth factors, antibiotics, and FBS according to the recommended concentrations. Adjust the customized mix of growth factors for primary cells.
3. Cell Seeding:
   - After sorting, determine the concentration and viability of the sorted epithelial cells using a hemocytometer or automated cell counter.
   - Seed the sorted cells into pre-coated culture vessels at the desired density (usually between 5,000 to 10,000 cells/cm²) in the culture medium. Maintain appropriate volumes based on the vessel size.
4. Cell Culture and Maintenance:
   - Place the culture vessels in a cell culture incubator maintained at 37°C with 5% CO2 and high humidity.
   - Regularly check the cells under a microscope to monitor their growth, morphology, and confluency. Change the medium every 48-72 hours or as needed, depending on the cell growth rate. Fig. 7 shows first attached cells after sorting.
5. Passaging the Cells:
   - Once the cells reach 70-80% confluency, they should be passaged to avoid overgrowth.
   - Rinse the cells with DPBS to remove residual medium and cellular debris.
   - Add trypsin-EDTA solution to detach the cells from the culture vessel. Incubate for a few minutes at 37°C until cells detach.
   - Neutralize trypsin activity by adding an appropriate volume of culture medium containing FBS.
   - Centrifuge the cells at low speed (300-400 × g) for 5 minutes to pellet them.
   - Resuspend the cells in fresh culture medium and seed them into new culture vessels at the desired density.
6. Experimental Use or Downstream Applications:
   - Use the cultured epithelial cells for downstream applications such as functional assays, gene expression analysis, characterization, once they reach the desired passage and confluency.

### 4. Characterization of isolated cell line

These protocols offer a comprehensive approach to characterizing the isolated epithelial cells by assessing gene expression, protein expression, and immunofluorescence using markers such as Casein genes, Lactose, and Cytokeratins (CK18). Adjustments can be made based on specific antibodies, primers, or reagents used in your laboratory or experimental setup.

### Gene Expression Analysis

### Materials:

- Isolated epithelial cells
- TRIzol reagent
- Chloroform
- Isopropanol
- 75% ethanol
- DNase I (optional)
- Reverse transcriptase kit
- Real-time PCR machine
- Primers specific for milk component genes (e.g., Casein genes)

### Procedure:

1. RNA Extraction:
   - Lyse the isolated cells in TRIzol reagent following the manufacturer's instructions.
   - Add chloroform, mix thoroughly, and centrifuge to separate the phases.
   - Collect the aqueous phase containing RNA, and precipitate RNA using isopropanol.
   - Wash RNA pellet with ethanol, air dry, and dissolve in RNase-free water.
2. cDNA Synthesis:
   - Remove any genomic DNA contamination using DNase I (if required) following the manufacturer's protocol.
   - Synthesize complementary DNA (cDNA) using a reverse transcriptase kit according to the manufacturer's instructions.
3. Real-time PCR (qPCR):
   - Set up qPCR reactions using cDNA, gene-specific primers, and a suitable master mix.
   - Run qPCR cycles in a real-time PCR machine according to the optimized cycling conditions.
   - Analyze the expression levels of milk component genes relative to housekeeping genes or controls.

### Protein Expression Analysis

### Materials:

- Isolated epithelial cells
- Cell lysis buffer
- Protein assay kit (e.g., BCA assay)
- SDS-PAGE gels
- Western blotting apparatus
- Primary antibodies against milk proteins (e.g., Lactose, Casein)
- Secondary antibodies conjugated with HRP (Horseradish Peroxidase)
- Chemiluminescent substrate

### Procedure:

1. Protein Extraction:
   - Lyse the isolated cells in cell lysis buffer containing protease inhibitors.
   - Centrifuge lysates to collect the protein-containing supernatant.
2. Protein Quantification:
   - Quantify protein concentration in the lysates using a protein assay kit (e.g., BCA assay).
3. SDS-PAGE and Western Blotting:
   - Prepare SDS-PAGE gels and load protein samples after denaturation.
   - Run electrophoresis and transfer proteins onto a membrane using a Western blotting apparatus.
4. Immunoblotting:
   - Block the membrane and incubate with primary antibodies specific to milk proteins (e.g., Lactose, Casein).
   - Wash and incubate the membrane with secondary antibodies conjugated with HRP.
   - Detect protein bands using a chemiluminescent substrate and expose to X-ray film or use a chemiluminescent imaging system.

### Immunofluorescence Analysis:

### Materials:

- Isolated epithelial cells (grown on coverslips)
- Fixative (e.g., paraformaldehyde)
- Permeabilization buffer (e.g., Triton X-100)
- Primary antibodies against Cytokeratins (e.g., CK18)
- Fluorescently labeled secondary antibodies
- DAPI (4',6-diamidino-2-phenylindole) for nuclear staining
- Mounting medium

### Procedure:

1. Sample Preparation:
   - Fix isolated cells with paraformaldehyde, permeabilize with Triton X-100, and block non-specific binding.
2. Immunostaining:
   - Incubate cells with primary antibodies against Cytokeratins (e.g., CK18) overnight at 4°C.
   - Wash cells and incubate with fluorescently labeled secondary antibodies for 1 hour at room temperature.
   - Stain nuclei with DAPI (1:1000 dilution) for 5 minutes.
3. Mounting and Imaging:
   - Mount coverslips with cells using a suitable mounting medium.
   - Visualize immunofluorescence signals using a fluorescence microscope or confocal microscope.

### 5. Immortalization of isolated and characterized cell lines

Immortalization techniques offer potential strategies to immortalize milk-derived epithelial cells by preventing cellular senescence, resetting cellular aging mechanisms, or modifying senescence-related genes while aiming to retain the cells' ability to produce milk components. Different methods can be employed such as:

### 5.1 Telomerase Activation:

Overview: Telomeres, the protective caps at the ends of chromosomes, shorten with each cell division, eventually leading to cellular senescence. Activation of telomerase, an enzyme that maintains telomere length, can prevent or delay cellular aging and senescence.

Procedure:
- Introduce exogenous telomerase (hTERT) expression into the cells using viral vectors or other gene delivery systems.
- Continuous expression of telomerase can elongate telomeres, extending the replicative lifespan of the cells and potentially immortalizing them.

### 5.2 Induced Pluripotent Stem Cells (iPSCs) Differentiation:

Overview: Reprogramming differentiated cells, such as milk epithelial cells, into induced pluripotent stem cells (iPSCs) can reset their cellular clock, allowing for indefinite self-renewal and differentiation potential.

Procedure:
- Reprogram the isolated cells into iPSCs by inducing the expression of pluripotency-associated transcription factors (e.g., OCT4, SOX2, KLF4, and c-MYC).
- After successful reprogramming, differentiate iPSCs back into milk-producing epithelial cells, creating a theoretically immortalized cell line with retained characteristics.

### 5.3 CRISPR/Cas9-Mediated Modifications of Senescence-related Genes:

Overview: Using CRISPR/Cas9 gene editing technology, specific modifications can be made to genes associated with senescence pathways, allowing cells to bypass or delay the senescence process while retaining their milk-producing characteristics.

Procedure:
- Identify and target senescence-related genes using CRISPR/Cas9 to introduce precise modifications (e.g., knockout or knock-in mutations).
- Modify genes involved in cell cycle regulation, DNA damage response, or other pathways related to cellular aging, aiming to extend the replicative lifespan of the cells without altering their milk-producing traits.

## Claims

1. Method for preparing and/or isolating a cell population being enriched in lactating cells, wherein the method comprises the following steps:
(a) providing (a-1) milk or (a-2) a mammary tissue;
(b) selecting a cell population comprising lactating cells from the milk or from the mammary tissue, wherein the lactating cells to be enriched are selected on the basis of at least one cell marker selected from the group consisting of:
CD49f, EpCAM/CD326, Cytokeratin 7, Cytokeratin 8, Cytokeratin 10, Cytokeratin 18, Cytokeratin 19, Muc1/CD227, CD24, CD133/ PROM1, Her2, CD117, CD166, CD282, CD340, Claudin 4, C-MET, ER, PR, VDR;
and optionally further one or more selected from CD9, CD47, CD54, CD59, CD95, CD164, CD49b, CD66, CK8/18, CK19, MUC1, EPCAM, CD13, CD15s/CD73/CLA, CD166, CD227, CD340, CD10, CD90, CD200, CD29, CD142, CD271, CK5, CK14, CK17, SMA, V1M, CD34/CD39/CD140b, CD49e, Claudin-4, E-Cadherin, EGFR, Integrin α9β1, Nectin-4; and
(c) obtaining a cell population being enriched in lactating cells, wherein the cell population comprises lactating cells in an amount of at least 70% based on the total amount of cells being present in the cell population.

2. Method according to claim 1, wherein in step (b) the lactating cells to be enriched are selected on the basis of at least one cell marker selected from the group consisting of:
CD49f, EpCAM/CD326, Cytokeratin 7, Cytokeratin 8, Cytokeratin 10, Cytokeratin 18, Cytokeratin 19, Muc1/CD227, CD24, CD133/ PROM1, Her2, CD117, CD166, CD282, CD340, Claudin 4, C-MET, ER, PR, VDR;
and optionally further one or more selected from CD9, CD47, CD54, CD59, CD95, CD164, CD49b, CD66, CK8/18, CK19, MUC1, EPCAM, CD13, CD15s/CD73/CLA, CD166, CD227, CD340, CD10, CD90, CD200, CD29, CD142, CD271, CK5, CK14, CK17, SMA, V1M, CD34/CD39/CD140b, and CD49e.

3. Method according to claim 1 or 2, wherein in step (b) the lactating cells to be enriched are selected on the basis of at least one cell marker selected from the group consisting of CD49f, EpCAM, CD24, CD29, CD10, and CK18,
and optionally further one or more selected from CD9, CD47, CD54, CD59, CD95, CD164, CD49b, CD66, CD24, CK8/18, CK19, MUC1, EPCAM, CD13,
CD15s/CD73/CLA, CD166/CD227/CD340, CD10, CD90, CD200, CD29, CD10, CD142, CD271, CK5, CK14, CK17, SMA, V1M, CD34/CD39/CD140b, and CD49e.

4. Method according to claim 1, wherein in step (b) the lactating cells to be enriched are selected on the basis of at least one cell marker selected from the group consisting of:
CD49f, CD326 (EpCAM), CD24, CD133/PROM1, CD24, Muc1, CD29, CD227, CD166, CD10, CD282, CD340;
preferably selected from the group consisting of CD49f, EpCAM, CD24, and CD29, CD10;
and optionally further
Claudin 4, CD9, CD47, CD54, CD59, CD95, CD49b, CD13, CD166, CD227, CD340, CD90, CD200, CD142, CD271, CD34, CD39, CD140b, E-Cadherin, Claudin 4, EGFR, Integrin α9β1, Nectin-4, Her2, CD117, and CD49e.

5. Method according to claim 4, wherein in step (b) the lactating cells to be enriched are further selected on the basis of at least one cell marker selected from the group consisting of CK7, CK8, CK10, CK18, CK19, smooth muscle actin (SMA), vimentin (V1M), Hormone receptors such as ER (estrogen receptor), PR (progesterone receptor), and VDR (vitamin D receptor).

6. Method according to claims 1, 2, 3, and 5, wherein in step (b) the lactating cells to be enriched are further selected on the basis of at least one surface marker selected from the group consisting of
CD24 positive and CD29 negative;
and optionally being further CD49f positive or CD49f negative; EpCAM positive or EpCAM negative; CK18 positive and CD10 negative.

7. Method according to any one of the preceding claims, wherein in step (b) the lactating cells to be enriched are selected based on the following surface marker pattern:
(i) CD24 medium expression;
CD49f positive; and
EpCAM negative;
and/or
(ii) CD24 high;
CD49f negative;
CD29 low;
EpCAM positive;
CD10 negative;
and/or
(iii) CD24 medium expression;
CD49f negative;
CD29 low;
EpCAM positive,
optionally wherein the cell population has less than 30 % cells that exhibit the following surface maker pattern:
(iv) CD24 negative;
CD49f high;
CD29 high;
EpCAM negative
DC10 high.

8. Method according to any of the preceding claims, wherein the milk of step (a) is cow milk, preferably obtained from a cow in a status range between day 30 and day 250 of lactation, more preferably between day 30 and day 150 of lactation, and even more preferably between day 90 and day 150 of lactation.

9. Method according to any one of the preceding claims, wherein the cell population comprises lactating cells in an amount of at least 70%, preferably at least 80%, more preferably in an amount of at least 90%, more preferably in an amount of at least 95%, and most preferred in an amount of at least 98%, based on the total cells of the cell population.

10. Method according to any one of the preceding claims, wherein the cell population obtained in step (c) is a second cell population, and wherein step (b) comprises at least one pre-selection step (b-0), thereby first obtaining a first cell population, wherein the ratio of lactating cells to non-lactating cells in the first cell population after the pre-selection step is enhanced compared to the ratio of lactating cells to non-lactating cells prior to the pre-selection step.

11. Cell culture comprising a cell population, wherein this cell population comprises lactating cells in an amount of at least 70% based on the total cells of the cell culture, and wherein lactating cells are characterized based on the presence or absence of at least one cell marker selected from the group as defined in step (b) of claims 1 to 9.

12. Cell culture according to claim 11, wherein this cell population comprises lactating cells in an amount of at least 70% based on the total cells of the cell culture, and wherein lactating cells are characterized based on the presence or absence of at least one cell marker selected from the group consisting of CD49f, EpCAM/CD326, Cytokeratin 7, Cytokeratin 8, Cytokeratin 10, Cytokeratin 18, Cytokeratin 19, Muc1/ CD227, CD24, CD133/ PROM1, Her2, CD117, CD166, CD282, CD340, Claudin 4, C-MET, ER, PR ,VDR;
and optionally further CD9, CD47, CD54, CD59, CD95, CD164, CD49b, CD66, CD24, CK8/18, CK19, MUC1, EPCAM, CD13, CD15s/CD73/CLA, CD166/CD227/CD340, CD10, CD90, CD200, CD29, CD10, CD142, CD271, CK5, CK14, CK17, SMA, V1M, CD34/CD39/CD140b, and CD49e;
more preferably,
the lactating cells are characterized based on the expression or non-expression of cell marker being selected from the group consisting of CD49f, EpCAM, CD24, CD29, CD10, and CK18;
and even more preferablythe lactating cells are **characterized by** a cell marker status being CD24 positive and CD29 negative;
and optionally being further CD49f positive or CD49f negative; EpCAM positive or EpCAM negative; CK18 positive and CD10 negative;
and optionally,
the cell culture is further **characterized by** a receptor status of being estrogen receptor alpha (ERα) positive and/or progesterone receptor (PR) positive.

13. Cell culture according to claim11 or 12, wherein the cell population comprises lactating cells in an amount of at least 75%, preferably at least 80%, more preferably at least 90%, even more preferably in an amount of at least 95%, and most preferred in an amount of at least 98%, based on the total cells of the cell culture.

14. Cell culture according to any one of claims 11 to 13, wherein the cell population comprises cells that exhibit the following cell maker pattern:
(i) CD24 medium expression;
CD49f positive; and
EpCAM negative;
and/or
(ii) CD24 high;
CD49f negative;
CD29 low;
EpCAM positive;
CD10 negative;
and/or
(iii) CD24 medium expression;
CD49f negative;
CD29 low;
EpCAM positive,
optionally wherein the cell population has less than 30% cells that exhibit the following surface maker pattern:
(iv) CD24 negative;
CD49f high;
CD29 high;
EpCAM negative
DC10 high.

15. Method of in vitro milk production, comprising the following steps:
(i) providing the cell population as defined in any one of claims 11 to 14, or providing the cell population being enriched in lactating cells by the method as defined in any one of claims 1 to 10;
(ii) cultivating the cell population of step (i) under suitable conditions; and
(iii) harvesting a milk product.
